# EUROPEAN PATENT SPECIFICATION

(11) **EP 0 240 208 B1**
(45) Date of publication and mention of the grant of the patent: **17.11.1994**
(21) Application number: 87302367.5
(22) Date of filing: 19.03.1987
(51) Int. Cl.: C12N 15/05, C12N 15/55, A01H 1/00

(54) **Anti-sense regulation of gene expression in plant cells**
Anti-sense-Regulierung von Genexpression in Pflanzenzellen
Réglage anti-sens d'expression de gènes dans les cellules végétales

(30) Priority: 28.03.1986 US 845676; 17.10.1986 US 920574
(43) Date of publication of application: 07.10.1987
(60) Divisional application: 91109006.6
(73) Proprietor: CALGENE, INC., Davis California 95616 (US)
(72) Inventor: Shewmaker, Christine K., Davis, California 95616 (US); Kridl, Jean C., Davis, California 95616 (US); Hiatt, William R., Davis, California 95616 (US); Knauf, Vic, Davis, California 95616 (US)
(74) Representative: Harrison, David Christopher

(56) References cited:
- EP-A- 0 159 779
- EP-A- 0 223 399
- EP-A- 0 223 452
- EP-A- 0 240 332
- EP-A- 0 271 988
- WO-A-86/05516
- WO-A-88/01645
- CHEMICAL ABSTRACTS, vol. 107, 1987, page 388, no. 74243f, Columbus, Ohio, US;D. GRIERSON et al.: "Expression and function of ripening genes", &PLANT BIOL. 1987, 4(TOMATO BIOTECHNOL.), 309-23
- CHEMICAL ABSTRACTS, vol. 103, 1985, page 211, no. 155198q, Columbus, Ohio, US;P.E. MANSSON et al.: "Characterization of fruit-specific cDNAs from tomato",& MOL. GEN. GENET. 1985, 200(3), 356-
- NATURE, vol. 315, 13th June 1985, pages 601-603; J. COLEMAN et al.: "A novel immune system against bacteriophage infection using complementary RNA (micRNA)", p. 603
- CHEMICAL ABSTRACTS, vol. 101, 1984, page 416, no. 127096v, Columbus, Ohio, US;G.E. HOBSON et al.: "The inhibition of tomato fruit ripening by silver",& J. PLANT PHYSIOL. 1984, 116(1), 21-9
- BIO/TECHNOLOGY, June 1984, pages 520-527, Ciba-Geigy Corp., US; G. HELMER etal.: "A new chimeric gene as a marker for plant transformation: the expressionof Escherichia Coli Beta-galactosidase in sunflower and tobacco cells"
- PROCEEDINGS OF THE NATIONAL ACADEMY OF SCIENCES OF THE USA, vol. 83, no. 15,August 1986, pages 5372-5376, Washington, US; J.R. ECKER et al.: "Inhibition ofgene expression in plant cells by expression of antisense RNA"
- J. CELL BIOCHEM., vol. 0, no. 10, part C, 1986, page 41, no. J108; L.S. LOESCH-FRIES et al.
- CHEMICAL ABSTRACTS, vol. 109, 1988, page 400, no. 125987c, Columbus, Ohio, US;P.H. MORGENS et al.: "Searching for molecular mechanisms involved in fruitripening",
- SCIENCE, vol. 229, 26th July 1985, pages 345-352; J.G. IZANT et al.:"Constitutive and conditional suppression of exogenous and endogenous genes byanti-sense RNA"
- CHEMICAL ABSTRACTS, vol. 104, 1986, page 159, no. 15979r, Columbus, Ohio, US;A. SLATER et al.: "Isolation and characterization of cDNA clones for tomatopolygalacturonase and other ripening-related proteins",& PLANT MOL. BIOL. 1985, 5(3), 137-147
- CHEMICAL ABSTRACTS, vol. 97, 1982, page 456, no. 88858x, Columbus, Ohio, US; R.
- PRESSEY et al.: "Pectic enzymes in 'Long Keeper' tomatoes", &HORTSCIENCE 1982, 17(3, Sect. 1), 398-400
- Grierson et al. Nucleic Acids Res. 14(21): 8595-8603 (1986)
- DellaPenna et al, Proc. Natl. Acad. Sci. 83: 6420-6424 (1986)
- Giovannoni et al., The Plant Cell 1: 53-63 (1989)
- Grierson et al. Phil. Trans. R. Soc. B 314: 399-410 (1986)

## Description

The modification of plants by genetic engineering has lagged behind the understanding and utilization of the molecular biology of unicellular organisims and mammalian cells. Techniques that have proven effective for stable transformation of unicellular microorganisms or mammalian cells with foreign DNA have not found useful analogy with plant cells. Therefore, despite the many achievements involved with unicellular microorganisms and mammalian cells, the number of achievements with plant cells has been substantially fewer and the experience with the other types of organisms has not been readily translatable into successful practices with plant cells.

In many situations it will be desirable to modify an existing trait of a plant cell, rather than introduce a new trait. Thus, one may wish to modify the activity of a particular enzyme, provide for the preferential expression of one allele as compared to another, one isozyme as compared to another, or the like. In many instances one may only wish to reduce the amount of expression of a structural gene, rather than inhibit expression entirely. It is therefore of interest to develop techniques which will allow for directed modification of the phenotype of particular plant cells, plant tissues or plants.

Crowley et al. Cell (1985) 43:633-641, describe the use of an anti-sense construct of the discoidin gene transfected into Dictyostelium to repress expression of endogenous discoidin genes. See also references cited therein. Anti-sense regulation has also been described by Rosenberg et al. Nature (1985) 313:703-706; Preiss et al. Nature (1985) 313:27-32; Melton, Proc. Natl. Acad. Sci. USA (1985) 82:144-148; Izant and Weintraub, Science (1985) 229:345-352; and Kim and Wold, Cell (1985) 42:129-138. See also, Izant and Weintraub Cell (1984) 36:1007-1015; Pestka et al. Proc. Natl. Acad. Sci. USA (1984) 81:7525-7528; Mizuno et al. ibid (1984) 81:1966-1970; Coleman et al. Cell (1984) 37:683-691; Travers, Nature (1984) 311:410 and Weintraub et al. Trends in Genetics (1985) 1:22-25. McGarry and Lindquist, Proc. Natl. Acad. Sci. USA (1986) 83:399-403, report the inhibition of heat shock protein synthesis by heat inducible anti-sense RNA.

### SUMMARY OF THE INVENTION

Regulation of polygalacturonase (PG) expression in plant cells is achieved by integrating into the plant cell host a DNA sequence comprising a gene in which the transcribed DNA sequences are at least partially complementary to a PG DNA sequence already transcribed by the host. The exogenous integrated DNA will be under the transcriptional control of a transcriptional initiation region recognized by the plant cell host. Transcription of the exogenous integrated DNA will result in multicopies of an anti-sense RNA which will be complementary to endogenous PG RNA of she host cell. This anti-sense mRNA will result in reduction of the functioning of the naturally existing PG RNA.

In providing the means for attaining the foregoing result, the present invention also provides PG gene sequences, as taught and enabled herein.

### BRIEF DESCRIPTION OF THE DRAWINGS

Fig. 1 indicates the EcoRI-BamHI fragment from pCGN 1401. This fragment corresponds to the 5'-portion of P1 including the region encoding the N-terminus of the mature polygalacturonase protein. The underlined amino acids are predicted from the DNA sequence and agree with the amino acid sequence determined by chemical sequencing from purified polygalacturonase; and
Fig. 2 is a flow chart of the various plasmids used in the construction of the binary vector pCGN783.

### DESCRIPTION OF THE SPECIFIC EMBODIMENTS

Methods and compositions are provided for modulating PG RNA utilization, particularly modulation of a PG-related phenotypic property of a plant host cell. The compositions involve transcription constructs having transcriptional initiation and termination regions separated by a sequence which is complementary to a sequence present on PG RNA, particularly messenger RNA, endogenous to the host. By an anti-sense means, various processes endogenous to the plant host cell may be modulated, so that the production of individual proteins may be reduced, multi-enzyme processes modulated, particular metabolic paths modulated or inhibited in preference to one or more other metabolic paths, production of non-proteinaceous products reduced, cell differentiation modified, and the like.

The sequence complementary to a sequence of the PG messenger RNA will usually be at least about 15 nucleotides, more usually at least about 20 nucleotides, preferably about 30 nucleotides, and more preferably about 50 nucleotides, and may be 100, or even 200, nucleotides or more, usually being fewer than about 5000 nucleotides, more usually being fewer than 2000 nucleotides, and preferably being fewer than 1000 nucleotides. The sequence may be complementary to any sequence of the messenger RNA, that is, it may be proximal to the 5'-terminus or capping site, downstream from the capping site, between the capping site and the initiation codon and may cover all or only a portion of the non-coding region, may bridge the non-coding and coding region, be complementary to all or part of the coding region, complementary to the 3'-terminus of the coding region, or complementary to the 3'-untranslated region of the mRNA.

In referring to Messenger RNA, the Messenger RNA may be processed or unprocessed, that is including introns Thus, the non-coding region may include the 5' or 3' non-coding flanking regions and the introns.

The particular site(s) to which the anti-sense sequence binds and the length of the anti-sense sequence will vary depending upon the degree of inhibition desired, the uniqueness of the sequence, the stability of the anti-sense sequence, or the like. Therefore, to some degree, these factors will be determined empirically based on the experience observed with a particular anti-sense sequence.

The sequence may be a single sequence or a repetitive sequence having two or more repetitive sequences in tandem, where the single sequence may bind to a plurality of messenger RNAs.

The transcriptional construct will be comprised of, in the direction of transcription, a transcriptional initiation region, the sequence coding for the anti-sense RNA on the sense strand, and a transcriptional termination region.

The transcriptional initiation region may provide for constitutive expression or regulated expression. A large number of promoters are available which are functional in plants. These promoters may be obtained from Ti- or Ri-plasmids, from plant cells, plant viruses or other hosts where the promoters are found to be functional in plants. Illustrative promoters include the octopine synthetase promoter, the nopaline synthase promoter, the manopine synthetase promoter, etc., as illustrative of promoters of bacterial origin functional in plants. Viral promoters include the cauliflower mosaic virus full length (35S) and region VI promoters, etc. Endogenous plant promoters include the ribulose-1,6-biphosphate (RUBP) carboxylase small subunit (ssu), the β-conglycinin promoter, the phaseolin promoter, the ADH promoter, heat-shock promoters, tissue specific promoters, e.g., promoters associated with fruit ripening, etc.

The transcriptional initiation region may be a naturally-occurring region, a RNA polymerase binding region freed of the regulatory region, or a combination of an RNA ploymerase binding region from one gene and regulatory region from a different gene. The regulatory region may be responsive to a physical stimulus, such as heat, with heat shock genes, light, as with the RUBP carboxylase SSU, or the like. Alternatively, the regulatory region may be sensitive to differentiation signals, such as the β-conglycinin gene, the phaseolin gene, or the like. A third type of regulatory region is responsive to metabolites. The time and level of expression of the anti-sense RNA can have a definite effect on the phenotype produced. Thus the promoters chosen will determine the effect of the anti-sense RNA.

Any convenient termination region may be employed, conveniently the termination region of the RNA polymerase binding region, or a different termination region. Various termination regions are available and the choice is primarily one of convenience, where prior constructions or DNA sequences may be available. Conveniently, the opine termination regions may be employed, or termination regions from endogenous genes, such as the genes which have been described previously.

The various fragments may be joined by linkers, adapters, or the like, or directly where convenient restriction sites are available. The DNA sequences, particularly bound to a replication system, may be joined stepwise, where markers present on the replication system may be employed for selection.

The constructions of the subject invention may be introduced into the host cell in a variety of ways. Of particular interest is the use of A. tumefaciens, with protoplasts, injured leaves, or other explant tissues. Other techniques which may find use include electroporation with protoplasts, liposome fusion, microinjection, or the like. The particular method for transforming the plant cells is not critical to this invention.

Any plant containing PG may be employed in accordance with this invention, including angiosperms, gymnosperms, monocotyledons, and dicotyledons. Plants of interest for determination of PG include cereals such as wheat, barley, maize, triticale, etc.; fruits, such as apricots, oranges, grape-fruits, apples, pears, avocados, etc.; nuts, such as walnuts, almonds, filberts, pecans, etc.; vegetables, such as carrots, lettuce, tomatoes, celery, turnips, potatoes, broccoli, asparagus, etc.; woody species, such as poplar, pine, sequoia, cedar, oak, etc; ornamental flowers; or other cash crops, such as tobacco, jojoba, rapeseed, Cuphea, soybeans, sunflower, sugar beet, safflower, etc.

After the cells have been transformed, the cells will be regenerated into plants. Various techniques exist for regenerating plants from cells. Calli can be developed from the cells and the calli induced to form shoots which may then be transfered to an appropriate nutrient medium in soil to regenerate the plant. The plants will then grow and, as appropriate, may be crossed with other plants so as to establish the stability of the change in phenotype over a number of generations. Other techniques may be employed for regenerating the plants without pollination or fertilization. Because those plant genotypes that can be regenerated from culture may not be directly applicable as crop varieties, the transformed plant may be crossed with alternate untransformed germplasm in order to transfer the trait to appropriate breeding lines.

Modulation of PG expression can typically be used for delaying the ripening in fruits, such as tomato or avocado.

The transcription construct will usually be joined to a replication system, particularly a bacterial replication system, for manipulation and cloning during its construction. The replication system can be any convenient replication system, particularly one that is functional in E. coli, and one or more markers may be present for detecting transformed bacteria.

Where A. tumefaciens or A. rhizogenes is employed for transferring the DNA, the construction will also be joined to at least one T-DNA border. Thus, the construction will include one T-DNA border, particularly the right T-DNA border, or may be sandwiched between the left and right T-DNA borders.

Various techniques exist for transferring the construct employing the Ti- or Ri-plasmid as the means for the transfer. These techniques include providing for a plasmid which is capable of replication in Agrobacterium, where the construct in T-DNA becomes integrated into the Ti- or Ri-plasmid by recombination. Alternatively, binary vectors may be employed, where the Ti- or Ri-plasmid in the Agrobacterium may or may not have a T-DNA region homologous with the T-DNA of the construct. In either event, so long as the vir genes are present on the endogenous plasmid, the T-DNA can be transferred successfully to the plant.

By having a marker as part of the expression construct, particularly antibiotic resistance, such as kanamycin resistance, hygromycin resistance, gentamicin resistance, bleomycin resistance, etc., one can select for those plant cells which have retained the construct in functional form. Where binary vectors are being employed and where the T-DNA in the Ti- or Ri-plasmid of the Agrobacterium retains the oncogenes, one will select for morphologically normal cells, which lack oncogenic expression.

Where electroporaticn or microinjection is employed, there need be no concern about gall formation and one expects that the morphology of the resulting plants would be normal, except for the modified phenotype.

An example of the use of an anti-sense strand is the regulated modulation of the expression of polygalacturonase (PG) in tomatoes. The ability to reduce the production of polygalacturonase could have a positive effect on the solids content of the tomato plant and improve tomato processing.

To control polygalacturonase expression in tomato fruit a transcription construct is prepared having the anti-sense strand of the polygalacturonase gene transcribed. The entire gene including flanking regions need not be employed, conveniently cDNA or a fragment thereof may be employed. The fragment will be from about 100 to 2000nt, more usually from 150 to 1000nt.

The transcription initiation regulatory region is desirably inducible, rather than constituitive, particularly being active at the time of fruit breaking (shortly prior to ripening). For this purpose the polygalacturonase gene transcriptional initiation region may be employed or a transcriptional initiation region of another gene associated with the development of fruit during ripening.

The manner of construction of the transcription cassette need not be repeated here. Once the construct has been prepared, it is introduced into tomato plant cells in accordance with conventional ways, and plants regenerated from the cells.

The following example is offered by way of illustration and not by way of limitation.

### EXPERIMENTAL

### Materials and Methods

T4 ligase was from ProMega Biotech. Restriction enzymes, Klenow polymerase fragment, and Bal31 were from Bethesda Research Laboratories (BRL).

### Construction of the octopine cassette, pCGN451.

The ocs5'-ocs3' cassette, was inserted into a derivative of pUC8 (Vieira and Messing, Gene (1982) 19:259-268), where a XhoI linker (CCTCGAGG) was inserted at the HincII site and the EcoRI site removed by filling in with the Klenow fragment of DNA polymerase. The octopine synthetase cassette was prepared by linking the XhoI (15208) - BamHI (13774) fragment from the octopine Ti-plasmid pTiA6 (Currier and Nester (1976) J. Bact. 126:157-165; Thomashow et al. Cell (1980) 19:729-739) containing the T-DNA border to the cut-down BamHI (13774) to EcoRI (linker) fragment (the numbering is by Barker, et al. Plant Mol. Biol. (1983) 2:335-350, for the closely related Ti-plasmid pTi15955). The cut-down BamHI-EcoRI fragment was obtained by digesting an EcoRI (13362) to BamHI (13774) subclone of the T-region of pTiA6 with XmnI (13512), followed by resection with Bal31 exonuclease. EcoRI linkers (GGAATTCC) were added and EcoRI to BamHI fragments of approximately 130bp gel purified, cloned into M13mp9 and sequenced. A clone in which the EcoRI linker was inserted at 13642 between the transcription initiations point and the translation initiation codon was identified by comparison with the sequence of de Greve et al. J. Mol. Appl. Genet. (1982) 1:499-512.

The EcoRI cleavage site was at position 13639, downstream from the mRNA start site. The SmaI site at 11207 was converted to a XhoI site using oligonucleotide linkers (CCTCGAGG) and the 3' end of the octopine gene from the EcoRI (12823) to the converted XhoI site added to the cassette. The resulting expression cassette having the octopine synthetase 5'-region (15208-13639) and 3'-region (12823-11207) was then inserted into the XhoI site of the modified pUC8 to provide pCGN451.

### Polygalacturonase Anti-sense Construct.

### Bacterial Strains.

**Table I**

| Bacterial Stains | | |
|---|---|---|
| Escherichia Coli Designation | Phenotype | Origin/Reference |
| 7118 | Δlac | Vieira and Messing |
| | | Gene (1982) 19:259-258 |
| Y1088 | hsdR⁻ hsdM⁺ | Young and Davis |
| Y1090 | Δlon | PNAS USA (1983) 80:1194-1198 |
| C2110 | polA | Stalker et al. |
| | | PNAS USA (1983) 80:5500-5504 |

### Enzymes and Radioisotopes

All enzymes were obtained from commercial sources and used according to the manufacturer's suggestions. Radioisotopes were obtained from New England Nuclear.

### Isolation of poly(A)+RNA

Ripe fruit of tomato cv. CaliGrande was harvested and frozen in liquid N₂. Frozen tissue was ground in a mortar and pestle in liquid N₂, and the resulting powder was extracted by homogenization with a Brinkman polytron in buffer described by Facciotti et al. Bio/Technology (1985) 3:241-246. Total RNA was prepared as described by Colbert et al. Proc. Natl. Acad. Sci. USA (1983) 80:2248-2252.

Polysaccharides were precipitated from total RNA preparations with 40mM sodium acetate and 0.5 vol ethanol (Mansson et al. (1985) Mol.Gen.Genet. (1985) 200:356-361. Poly(A)+RNA was isolated as described by Maniatis et al. (1982) Molecular Cloning: A Laboratory Manual, Cold Spring Harbor, New York.

### Synthesis of cDNA

Synthesis of cDNA from poly(A)+RNA was performed as described by Gubler and Hoffman, Gene (1983) 25:263-269 with the following modifications: The reaction mixture for synthesis of the first strand included 1mM dGTP, 1mM dATP, 1mM TTP, 0.5mM dCTP, 0.5 unit/µl RNasin (Promega), 4µg of tomato poly(A)+RNA, and 80-100 units of reverse transcriptase (Life Sciences). The reaction was stopped with 2µl of 500mM EDTA, then precipitated with 10µg tRNA, l vol 4M NH₄OAc, and 2.5 vol of ethanol overnight on dry ice.

Second strand synthesis was performed from approximately 500ng of the first strand reaction product. Aliquots of the first and second strand reaction mixtures were radiolabeled separately with 20µCi of 5'-[α -³²P] dCTP to monitor each reaction independently.

### Cloning of Double-Stranded cDNA in λgt11.

The double-stranded cDNA was EcoRI methylated as described by the manufacturer (New England Biolabs). After ethanol precipitation, the cDNA ends were blunted using 3 units of the Klenow fragment of DNA polymerase I (Bethesda Research Laboratories) the following conditions: 66mM Tris-HCl pH 7.5, 20mm MgCl₂, 100mM dithiothreitol, 100µM dGTP, dATP, TTP, and dCTP at room temperature for 1 hr. The DNA was then ethanol precipitated. After blunting, 2µg of EcoRI phosphorylated linkers were added to the cDNA in 10µl of ligase buffer (50mM Tris, pH 7.5, 10mM MgCl₂, 20mM dithiothreitol, 1mM ATP, and 5mg/ml bovine serum albumin). T₄ DNA ligase (1 Weiss unit, Weiss, J. Biochem. (1968) 243:4543, Promega) was added and incubated for 6 hr at 15°C. An additional Weiss unit of T₄ DNA ligase in 10µl of ligase buffer was then added and incubated for 24 hr at 15-19°C. The reaction was phenol extracted, ethanol precipitated and digested with 100 units EcoRI (New England Biolabs) for 6-8 hrs, phenol extracted and ethanol precipitated. Excess linkers and cDNA fewer than 500 base pairs were removed by chromatography on Bio-gel A-50m (100-200 mesh) and the sized cDNA was ligated to EcoRI-cleaved λgt11 vector DNA (Statagene) as described by Huynh et al. in DNA Cloning: A Practical Approach, ed. D.M. Glover, pp. 49-78, IRL Press, Oxford, England, 1985.

In vitro packaging reactions were performed with Giga-pack extracts (Stratagene) as described by the vendor. Initial test ligations and in vitro packaging were done using various dilutions of cDNA to empirically determine the optimal ration of cDNA/vector for production of recombinant phage. The packaged λgtll phage were plated on E. Coli Y1088 in the presence of isopropoyl-1-thio-β-D-galactoside (IPTG) and 5-bromo-4-cloro-3-indolyl-β-D-galactoside (X-gal) as described by Huynh et al. (1985), supra to determine the number of recombinants. Greater than 5x10⁶ recombinants at a 90% insertion rate was obtained in λgtll.

### Library Screening

Approximately 200,000 phage from an unamplified λgtll library were screened at a density of 20,000 plaques-forming units per 9cm square plate using E. coli Y1090 as the host as described by Huynh et al. (1985), except that NZY media (per liter: 5g NaCl, 2g MgCl₂, 10g NZamine type A (Sheffield Products), 5g yeast extract and 15g agar) was used. Plates were incubated and overlaid with nitrocellulose sheets containing IPTG as described by Huynh et al. (1985), supra. The nitrocellulose sheets were saturated with 0.5M Tris pH 8.0, 0.15M NaCl, 0.02% NaN₃, 0.1% Triton X-100 and 5% non-fat dry milk, then incubated 30 min at room temperature with the same buffer containing anti-polygalacturonase2 antibody (see below) diluted 1:1000. Bound antibody was detected with an alkaline phosphatase-conjugated second antibody (Promega) as described by the vendor. Positive plaques were purified by successive plating and phage DNA was prepared as described (Maniatis et al. (1982).

### Subcloning and Sequencing of cDNA Insert P1

Phage DNA from positive plaque P1 was digested with EcoRI and the resulting fragment was subcloned in EcoRI-digested vector M13 Blue Scribe Minus (Strata-gene) by in vitro ligation. Initial DNA sequencing was performed using single-stranded template from the Blue Scribe construct prepared as described by the manufacturer. All DNA sequencing was performed as described by Sanger et al., Proc. Natl. Acad. Sci. USA (1977) 74:5463 or Maxam and Gilbert, Methods Enzymol. (1980) 65:499-580. Overlapping sequences were obtained by subcloning purified BamHI-EcoRI, HinDIII-EcoRI, and BamHI-HinDIII fragments (Maniatis et al., supra) from the Blue Scribe construct into M13mp18 (Yanisch-Perron et al. Gene (1985) 53:103-119) and M13mp19 (Norrander et al. Gene (1983) 26:101-106).

### Polygalacturonase Purification for Protein Sequencing

Total cell wall bound proteins were prepared from ripe fruit of cv. CaliGrande as described by Crookes and Grierson, Plant Physiol. (1983) 72:1088-1093. The extract was dialyzed against 0.025M ethanolamine, pH 9.4, and applied to a 9 x 300mm column of chromatofocusing exchanger PBE 94 (Pharmacia) equilibrated with 0.025M ethanolamine, pH 9.4. Bound proteins were eluted with Polybuffer 96, pH 8.0 (Pharmacia). Fractions containing polygalacturonase were pooled and precipitated with ammonium sulphate (90% saturation) and further fractionated by chromatography over a hydroxyapatite (HAPT) HPLC column. Two ml volumes were layered onto the column and chromatographed at 1 ml/min using a linear gradient extending from 10mM to 350mM sodium phosphate, pH 6.8. Samples were monitored at A₂₈₀ and fractionated into 0.5ml volumes. Fractions collected from numerous runs which contained polygalacturonase were pooled and dialyzed against 6% acetic acid, then lyophilized.

### Protein Sequencing

Polygalacturonase prepared as described above was sequenced intact with a Beckman 890 M Liquid Phase Amino Acid Sequencer. The following N-terminal sequence was obtained:
Gly-ile-lys-val-ile-asn.

### Polygalacturonase Purification for Antibody Production

Tomato cell wall bound proteins were prepared from ripe fruit of cv. UC82B as described by Tucker and Grierson, Planta (1982) 155:64-67. The pellet from ammonium sulphate precipitation was dissolved in 150mM NaCl and then dialzyed overnight against the same buffer.

The protein solution was then fractionated on a TSK 3000/2000 HPLC sizing column using an isocratic gradient containing 10mM NaCl and 10mM Tris pH 7.2 at a flow rate of 0.5 ml/min.

TSK fractions containing polygalacturonase activity (Reisfeld et al. Nature (1962) 195:281-283) were pooled and and further fractionated over an hydroxyapatite HPLC column using a linear gradient of 10mM-350mM sodium phosphate, pH 6.8 and a flow rate of 1 ml/min. The peak containing polgalacturonase activity was collected and used to inject rabbits for antibody production.

Polygalacturonase for booster injections was prepared by resolving the cell wall bound protein preparation on SDS polyacrylamide gels. The material precipitated with ammonium sulphate (see above) was electrophoresed on 3mm thick and 14mm wide gels containing 12.5% polyacrylamide (Laemmli, Nature (1970) 227:680-685) and proteins were visualized by staining with Coomassie Brilliant Blue R. The region corresponding to the polygalacturonase bands (approximately 40,000 - 43,000 daltons) was excised, frozen, and ground with liquid N₂.

### Antibody Preparation

One rabbit was given 4 injections of polygalacturonase (125µg injection) over a one month period. The same rabbit was then given a booster injection of polygalacturonase (approximately 150µg) recovered from SDS polyacrylamide gels. An identical booster injection was again given one week after the first. The animal was exsanguinated 2 weeks later as a source of serum.

Six ml of the crude serum were diluted with 6ml of 0.1M sodium phosphate, pH 7.0, and applied to a 6ml column of Protein A-Sepharose (Sigma). The column was washed with 80ml of 0.1M sodium phosphate, pH 7.0, and the IgG fraction was then eluted with 0.1M glycine, pH 3.0. Fractions with the highest A280 were pooled, dialyzed against 20mM sodium phosphate pH 7.6, 150mM NaCl and concentrated on an Amicon XM80 membrane. Glycerol was then added to a final concentration of 40%.

Affinity purified antiserum was prepared by incubating the IgG fraction with polygalacturonase linked to a Tresacryl (Pharamacia) affinity chromatography matrix as described by the vendor. Polygalacturonase purified for protein sequencing was linked to 4ml of Tresacryl resin as described by the manufacturer. Five ml of IgG prepared as described above was diluted to 50ml with 0.01M Tris pH 7.5, 150mM NaCl and 0.1% Tween-20 (TBST) and incubated with the resin overnight at 4°C. The resin was then washed with TBST and eluted with 0.2M glycine, pH 2.75. Fractions with A280 were pooled and dialyzed against 10mM Tris pH 8.0, 150mM NaCl. The final volume of purified antibody was 12ml representing a 1:2 dilution of the original serum.

### RESULTS

### Identification of Polygalacturonase cDNAs

Twelve putative polygalacturonase clones were identified from the λgtll library by reaction with the antibody preparation described above. Using inserts purified from two of the clones as probes, Northern analysis demonstrated that one clone (C3) encoded mRNA expressed during tomato development in the manner and size expected for polygalacturonase mRNA.

To identify additional putative cDNA clones encoding polygalacturonase, phage DNA was prepared from the remaining 10 clones, digested with EcoRI and HindIII, and subjected to Southern blot hybridization analysis (Maniatis et al., supra) using clone C3 insert as a probe. An additional cDNA clone (P1) cross-hybridized to C3 and was further characterized to provide sequences for anti-sense expression. The identity of P1 as a polygalacturonase cDNA clone was confirmed by comparison of the amino acid sequence predicted from the DNA sequence to the actual polygalacturonase protein sequence. The clone encodes a portion of the polygalacturonase gene beginning approximately at the N-terminus of the mature polygalacturonase polypeptide and extending to the carboxy terminus including the 3' untranslated region.

### Construction of the Anti-sense Polygalacturonase Binary Plasmid

Phage P1 DNA was digested with EcoRI and the cDNA insert was ligated in EcoRI-digested M13 Blue Scribe Minus (Stratagene) to yield pCGN1401.

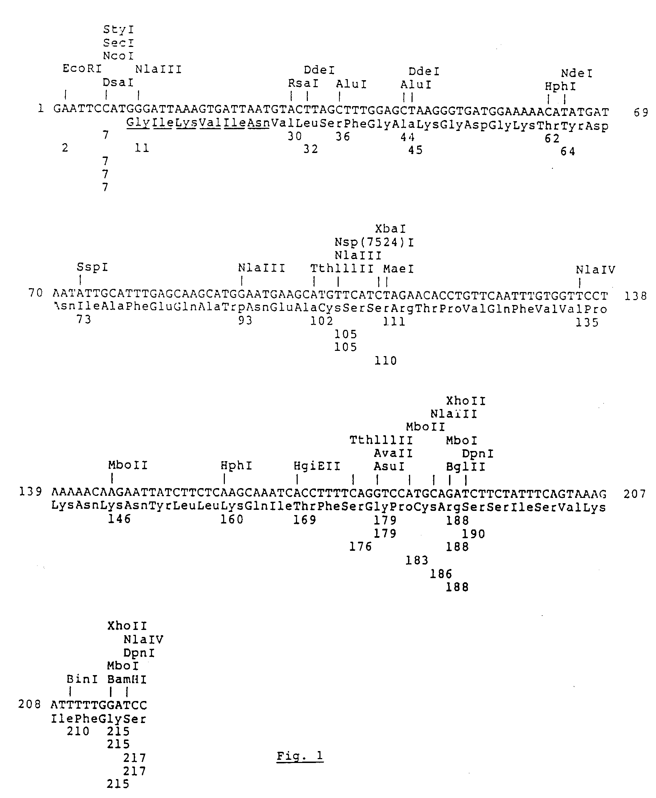

pCGN1401 was digested with BamHI and EcoRI to provide a 219 bp fragment (Fig. 1) which includes 7 bases (GAATTCC) of the EcoRI linker, 2 bases of the polygalacturonase leader sequence (AT), the triplet encoding the N-terminal amino acid of the mature polygalacturonase protein (GGG) and 210 additional bases to the BamHI site. This fragment was inserted in the unique BamHI-EcoRI site of the mas5'-ocs3' cassette, pCGN46 (Comai et al. Nature (1983) 317:741-744). This resulted in insertion of the fragment in the anti-sense (minus orientation) to the mas promoter to yield pCGN1402.

pCGN1402 was then digested with the restriction enzyme XhoI and cloned into the unique SalI site of the binary plasmid pCGN783 containing a plant kanamycin resistance marker between the left and right borders. This results in pCGN1403. This plasmid in E. coli C2110 was conjugated into Agrobacterium tumefaciens containing a disarmed Ti plasmid capable of transferring the polygalacturonase anti-sense cassette and the kanamycin resistance cassette into the plant host genome.

The Agrobacterium system which is employed is A. tumefaciens PC2760 (G. Ooms et al. Plasmid (1982) et al. Nature (1983) 303:179-181; European Patent Application 84-200239.6, 2424183).

### Construction of pCGN783

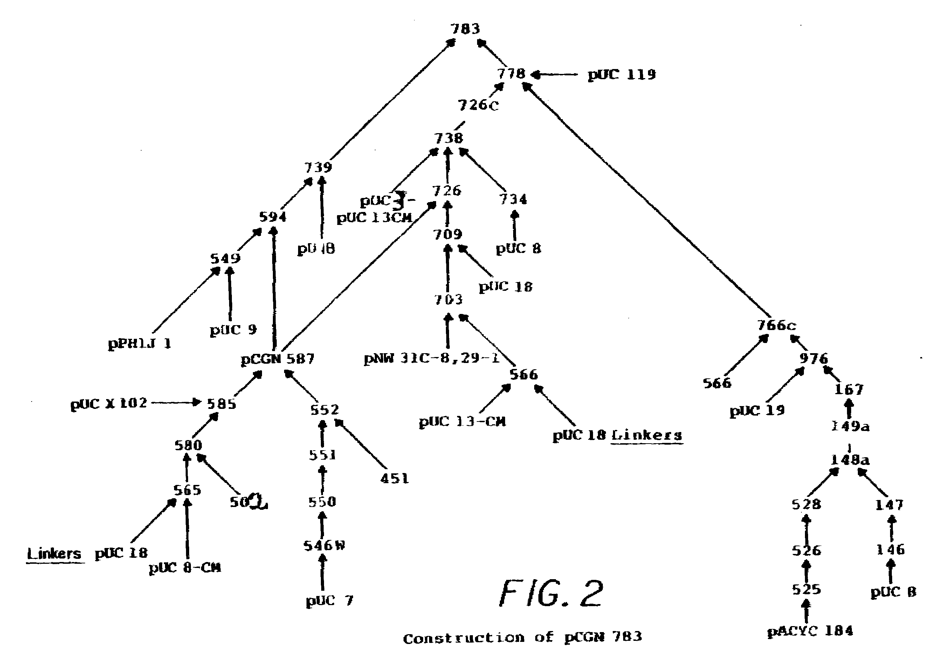

pCGN783 is a binary plasmid containing the left and right T-DNA borders of Agrobactrium tumefaciens octopine Ti-plasmid pTiA6 (Currier and Nester (1976) supra) the gentamycin resistance gene of pPH1JI (Hirsch et al. Plasmid (1984) 12:139-141), the 35S promoter of cauliflower mosaic virus (CaMV) (Gardner et al. Nucleic Acid Res. (1981) 9:1871-1880); the kanamycin resistance gene of Tn5 (Jorgensen, Mol. Gen. (1979) 177:65); and the 3' region from transcript 7 of pTiA6 (Currier and Nester (1976), supra). The construction of pCGN783 is outlined in Fig. 2.

### Construction of pCGN739 (Binary Vector)

To obtain the gentamicin resistance marker, the resistance gene was isolated from a 3.1kb EcoRI-PstI fragment of pPHIJI (Hirsch et al. 1984, supra) and cloned into pUC9 (Vieira et al. Gene (1982) 19:259-268) yielding pCGN549.

The pCGN549 HindIII-BamHI fragment containing the gentamicin resistance gene replaced the HindIII-BglII fragment of pCGN587 (for construction, see infra) creating pCGN594.

The pCGN594 HindIII-BamHI region which contains an ocs-kanamycin-ocs fragment was replaced with the HindIII-BamHI polylinker region from pUC18 (Yanisch-Perron, 1985, supra) to make pCGN739.

### Construction of 726c (1ATG-Kanamycin-3' region)

pCGN566 contains the EcoRI-HindIII linker of pUC18 (Yanisch-Perron, ibid) inserted into the EcoRI-HindIII sites of pUC13-cm (K. Buckley, Ph.D. thesis, UC-San Diego, 1985). The HindIII-BglII fragment of pNW31c-8, 29-1 (Thomashow et al. (1980) Cell 19:729) containing ORF1 and 2 (Barker et al. (1983), supra) was subcloned into the HindIII-BamHI sites of pCGN566 producing pCGN703.

The Sau3A fragment of pCGN703 containing the 3' region of transcript 7 from pTiA6 (corresponding to bases 2396-2920 of pTi15955 (Barker et al. (1983), supra) was subcloned into the BamHI site of puc18 (Yanisch-Perron et al. (1985), supra) producing pCGN709.

The EcoRI-SmaI polylinker region of pCGN709 was replaced with the EcoRI-SmaI fragment from pCGN587 (for production see infra) which contains the kanamycin resistance gene (APH3'II) producing pCGN726.

The EcoRI-SalI fragment of pCGN726 plus the BglII-EcoRI fragment of pCGN734 are inserted into the BamHI-SalI sites of pUC8-pUC13-cm (chloramphenical resistant, K. Buckley, PhD. Thesis, UC-San Diego, 1985) producing pCGN738. To construct pCGN734, the HindIII-SphI fragment of pTiA6 corresponding to bases 3390-3241 (Barker et al. (1983), supra) was cloned into the HindIII-SphI site of M13mp19 (Norrander et al. (1983), supra). Using an oligonucleotide corresponding to bases 3287 to 3300, DNA synthesis was primed from this template. Following S1 nuclease treatment and HindIII digestion, the resulting fragment was cloned into the HindIII-SmaI site of pUC19 (Yanisch-Perron et al. (1985), supra). The resulting EcoRI-HindIII fragment corresponding to bases 3287-3390 (Barker et al. (1983), supra) was cloned with the EcoRI to HindIII fragment of pTiA6 (corresponding to bases 3390-4494) into the EcoRI site of pUC8 (Vieira and Messing (1982), supra) resulting in pCGN734. pCGN726c is derived from pCGN738 by deleting the 900bp EcoRI-EcoRI fragment.

### Construction of pCGN766c (35s promoter - 3' region)

The HindIII-BamHI fragment of pCGN167 (for construction see infra) containing the CaMV-35S promoter, 1ATG-kanamycin gene and the BamHI fragment 19 of pTiA6 was cloned into the BamHI-HindIII sites of pUC19 (Norrander et al. (1983), supra; Yanisch-Perron et al. (1985), supra) creating pCGN976.

The 35S promoter and 3' region from transcript 7 was developed by inserting a 0.7kb HindIII-EcoRI fragment of pCGN976 (35S promoter) and the 0.5kb EcoRI-SalI fragment of pCGN709 (transcript 7:3', for construction, see supra) into the HindIII-SalI sites of pCGN566 creating pCGN766c.

### Final Construction of pCGN783

The 0.7kb HindIII-EcoRI fragment of pCGN766c (CaMV-35S promoter) was ligated to the 1.5kb EcoRI-SalI fragment of pCGN726c (1-ATG-KAN-3' region) into the HindIII-SalI sites of pUC119 (J. Vieira, Rutgers University, N. J.) to produce pCGN778.

The 2.2kb region of pCGN778, HindIII-SalI fragment containing the CaMV 35S promoter (1-ATG-KAN-3' region) replaced the HindIII-SalI polylinker region of pCGN739 to produce pCGN783.

pCGN587 was prepared as follows: The HindIII-SmaI fragment of Tn5 containing the entire structural gene far APH3'II (Jorgensen et al. Mol. Gen. (1979) 177:65), was cloned into pUC8 (Vieira and Messing, Gene (1982), 19:259), converting the fragment into a HindIII-EcoRI fragment, since there is an EcoRI site immediately adjacent to the SmaI site. The PstI-EcoRI fragment containing the 3'-portion of the APH3'II gene was then combined with an EcoRI-BamHI-SalI-PstI linker into the EcoRI site of pUC7 (pCGN546W). Since this construct does not confer kanamycin resistance, kanamycin resistance was obtained by inserting the BglII-PstI fragment of the APH3'II gene into the BamHI-PstI site (pCGN546X). This procedure reassembles the APH3'II gene, so that EcoRI sites flank the gene. An ATG codon was upstream from and out of reading frame with the ATG initiation codon of APH3'II. The undesired ATG was avoided by inserting a Sau3A-PstI fragment from the 5'-end of APH3'II, which fragment lacks the superfluous ATG, into the BamHI-PstI site of pCGN546W to provide plasmid pCGN550. The EcoRI fragment of pCGN550 containing the APH3'II gene was the then cloned into the EcoRI site of pUC8-pUC13 (K. Buckley (1985), supra) to give pCGN551.

Each of the EcoRI fragments containing the APH3'II gene was then cloned into the unique EcoRI site of pCGN451, which contains an octopine synthase cassette for expression, (as described in above) to provide pCGN548 (2ATG) and pCGN552 (1ATG). The plasmid pCGN451 having the ocs 5' and the ocs 3' in the proper orientation was digested with EcoRI and the EcoRI fragment from pCGN551 containing the intact kanamycin resistance gene inserted into the EcoRI site to provide pCGN552 having the kanamycin resistance gene in the proper orientation.

This ocs/KAN gene was used to provide a selectable marker for the trans type binary vector pCGN587.

The 5' portion of the engineered octopine synthase promoter cassette consists of pTiA6 DNA from the XhoI at bp 15208-13644 (Barker et al. (1983), supra), which also contains the T-DNA boundary sequence (border) implicated in T-DNA transfer. In the plasmid pCGN587, the ocs/KAN gene from pCGN552 provides a selectable marker as well the right border. The left boundary region was first cloned in M13mp9 as a HindIII-SmaI piece (pCGN502) (base pairs 602-2212) and recloned as a KpnI-EcoRI fragment in pCGN565 to provide pCGN580. pCGN565 is a cloning vector based on pUC8-Cm, but containing pUC18 linkers. pCGN580 was linearized with BamHI and used to replace the smaller BglI fragment of pVCK102 (Knauf and Nester, Plasmid (1982) 8:45), creating pCGN585. By replacing the smaller SalI fragment of pCGN585 with the XhoI fragment from pCGN552 containing the ocs/KAN gene, pCGN587 was obtained.

To construct pCGN167, the AluI fragment of CaMV (bp 7144-7735) (Gardner et al. Nucl. Acids Res. (1981) 9:2871-2888) was obtained by digestion with AluI and cloned into the HincII site of M13mp7 (Vieira Gene (1982) 19:259) to create C614. An EcoRI digest of C614 produced the EcoRI fragment from C614 containing the 35S promoter which was cloned into the EcoRI site of pUC8 (Vieira et al. Gene (1982) 19:259) to produce pCGN146.

To trim the promoter region, the BglII site (bp 7670) was treated with BglII and Bal31 and subsequently a BglII linker was attached to the Bal31 treated DNA to produce pCGN147.

pCGN148a containing a promoter region, selectable marker (KAN with 2 ATG's) and 3' region was prepared by digesting pCGN528 (see below) with BglII and inserting the BamHI-BglII promoter fragment from pCGN147. This fragment was cloned into the BglII site of pCGN528 so that the BglII site was proximal to the kanamycin gene of pCGN528.

The shuttle vector used for this construct, pCGN528, was made as follows. pCGN525 was made by digesting a plasmid containing Tn5 which harbors a kanamycin gene (Jorgenson et al. Mol. Gen. (1979) 177:65) with HindIII-BamHI and inserting the HindIII-BamHI fragment containing the kanamycin gene into the HindIII-BamHI sites in the tetracycline gene of pACYC184 (Chang & Cohen J. Bacteriol. (1978) 134:1141-1156). pCGN526 was made by inserting the BamHI fragment 19 9f pTiA6 (Thomashow et al. Cell (1980) 19:729-739) into the BamHI site of pCGN525. pCGN528 was obtained by deleting the small XhoI fragment from pCGN526 by digesting with XhoI and religating.

pCGN149a was made by cloning the BamHI kanamycin gene fragment from pMB9KanXXI into the BamHI site of pCGN148a.

pMB9KanXXI is a pUC4K variant (Vieira & Messing, Gene (1982) 19:259:268) which has the XhoI site missing but contains a functional kanamycin gene from Tn903 to allow for efficient selection in Agrobacterium.

pCGN149a was digested with BglII and SphI. This small BglII-SphI fragment of pCGN149a was replaced with the BamHI-SphI fragment from MI (see below) isolated by digestion with BamHI and SphI. This produces pCGN167, a construct containing a full length CaMV promoter, 1ATG-kanamycin gene, 3' end and the bacterial Tn903-type kanamycin gene. MI is an EcoRI fragment from pCGN550 (see construction of pCGN587) and was cloned into the EcoRI cloning site of M13mp9 in such a way that the PstI site in the 1ATG-kanamycin gene was proximal to the polylinker region of M13mp9.

The foregoing teaching makes available PG DNA, by the use of which it is possible to modulate expression of the PG gene in the genome of a plant host by providing for transcription of a sequence complementary to the messenger RNA of the PG gene expressed in the host.

pCGN1401 was deposited on 7th October 1986 at the A.T.C.C. and given Accession No. 67227.

## Claims

1. A recombinant DNA construct, comprising a polygalacturonase (PG) DNA sequence as found in the EcoRI insert of plasmid pCGN1401 (ATCC 67227), or as obtainable by the use of said insert DNA as a hybridization probe.

2. A recombinant DNA construct according to claim 1, wherein said DNA sequence comprises an open reading frame encoding PG.

3. A recombinant DNA construct according to claim 1 or claim 2 wherein said DNA sequence is a cDNA sequence derived from the PG gene.

4. A recombinant DNA construct according to any one of the preceding claims, which comprises also a prokaryotic replication system.

5. A recombinant DNA construct according to any one of the preceding claims including a marker for selection of a eukaryotic cell into which said construct has been inserted.

6. A recombinant DNA construct according to any one of the preceding claims wherein a sequence of at least 15 base pairs of PG DNA is joined, in the opposite orientation to normal for expression, to a transcription initiation region functional in plants, whereby an anti-sense PG RNA sequence will be transcribed in a plant cell operatively carrying said construct.

7. A recombinant DNA construct according to claim 6 wherein said DNA sequence comprises a strand complementary to at least 200 nucleotides of PG mRNA.

8. A recombinant DNA construct according to claim 6 or claim 7 including a transcription termination region functional in plants and located 3' to said PG DNA sequence.

9. A method for regulating the expression of a gene in a plant cell which comprises:
integrating into said plant cell genome a recombinant DNA construct of any one of claims 6, 7 and 8;
growing said plant cell containing said integrated construct, whereby said antisense RNA is transcribed and modulates the expression of PG in RNA in said plant cell.

10. A plant cell as obtainable by the method of claim 9.

11. A plant derived from a cell as obtainable by the method of claim 9.

12. The plasmid pCGN1401 (ATCC 67227).

## Patentansprüche

1. Rekombinantes DNA-Konstrukt, umfassend eine Polygalakturonase (PG)-DNA-Sequenz, wie in der EcoRI-Einfügung von Plasmid pCGN1401 (ATCC 67227) enthalten oder wie unter Verwendung dieser DNA-Einfügung als Hybridisierungssonde erhalten.

2. Rekombinantes DNA-Konstrukt nach Anspruch 1, worin die DNA-Sequenz einen für PG kodierenden, offenen Leserahmen umfaßt.

3. Rekombinantes DNA-Konstrukt nach Anspruch 1 oder 2, worin diese DNA-Sequenz eine aus dem PG-Gen abgeleitete cDNA-Sequenz ist.

4. Rekombinantes DNA-Konstrukt nach irgendeinem der vorangehenden Ansprüche, das auch ein prokaryotisches Replikationssystem umfaßt.

5. Rekombinantes DNA-Konstrukt nach irgendeinem der vorangehenden Ansprüche, das einen Marker zur Selektion einer eukaryotischen Zelle, in die dieses Konstrukt eingefügt wurde, enthält.

6. Rekombinantes DNA-Konstrukt nach irgendeinem der vorangehenden Ansprüche, worin eine Sequenz von zumindest 15 Basenpaaren der PG-DNA, in entgegengesetzter Ausrichtung als normalerweise zur Expression üblich, mit einem in Pflanzen funktionellen Transkriptionsinitiierungsbereich verbunden ist, wodurch in einer Pflanzenzelle, die dieses Konstrukt operativ trägt, eine anti-sense-PG-DNA-Sequenz transkribiert wird.

7. Rekombinantes DNA-Konstrukt nach Anspruch 6, worin die DNA-Sequenz einen zu zumindest 200 Nukleotiden von PG-mRNA komplementären Strang umfaßt.

8. Rekombinantes DNA-Konstrukt nach Anspruch 6 oder 7, umfassend einen in Pflanzen funktionellen und 3' dieser PG-DNA befindlichen Transkriptionsterminationsbereich.

9. Verfahren zur Steuerung der Expression eines Gens in einer Pflanzenzelle, das umfaßt:
Integrieren eines rekombinanten DNA-Konstrukts nach irgendeinem der Ansprüche 6, 7 oder 8 in das Genom der Pflanzenzelle;
Vermehren der das integrierte Konstrukt enthaltenden Pflanzenzelle, wodurch die antisense-RNA transkribiert wird und die Expression von PG in RNA in der Pflanzenzelle moduliert.

10. Nach dem Verfahren nach Anspruch 9 erhältliche Pflanzenzelle.

11. Pflanze, die von einer nach dem Verfahren nach Anspruch 9 erhältliche Zelle abgeleitet ist.

12. Plasmid pCGN1401 (ATCC 67227).

## Revendications

1. Construction d'ADN recombinant, comprenant une séquence d'ADN de polygalacturonase (PG) telle que trouvée dans l'insert de EcoRI du plasmide pCGN1401 (ATCC 67227) ou telle qu'on peut l'obtenir par l'utilisation dudit insert d'ADN en tant que sonde d'hybridation.

2. Construction d'ADN recombinant selon la revendication 1, où ladite séquence d'ADN comprend un cadre de lecture ouvert codant PG.

3. Construction d'ADN recombinant selon la revendication 1 ou 1a revendication 2, où ladite séquence d'ADN est une séquence d'ADNc dérivée du gène de PG.

4. Construction d'ADN recombinant selon l'une quelconque des revendications précédentes, qui comprend également un système de réplication procaryote.

5. Construction d'ADN recombinant selon l'une quelconque des revendications précédentes, comprenant un marqueur pour la sélection d'une cellule eucaryote dans laquelle ladite construction a été insérée.

6. Construction d'ADN recombinant selon l'une quelconque des revendications précédentes, où une séquence d'au moins 15 paires de bases de l'ADN de PG est jointe, à l'orientation opposée à la normale pour l'expression, à une région d'initiation de transcription fonctionelle dans les plantes, ainsi une séquence d'ARN de PG anti-sens sera transcrite dans une cellule de plante portant opérativement ladite construction.

7. Construction d'ADN recombinant selon la revendication 6, où ladite séquence d'ADN comprend un brin complémentaire sur au moins 200 nucléotides de l'ARNm de PG.

8. Construction d'ADN recombinant selon la revendication 6 ou la revendication 7, comprenant une région de terminaison de transcription fonctionnelle dans les plantes et placée côté 3' de ladite séquence d'ADN de PG.

9. Méthode pour la régulation de l'expression d'un gène dans une cellule de plante qui comprend :
l'intégration, dans ledit génome de la cellule de plante, d'une construction d'ADN recombinant selon l'une quelconque des revendications 6, 7 et 8 ;
la croissance de ladite cellule de plante contenant ladite construction intégrée, ce par quoi ledit ARN anti-sens est transcrit et module l'expression de PG dans l'ARN dans ladite cellule de plante.

10. Cellule de plante telle qu'elle est obtenue par la méthode de la revendication 9.

11. Plante dérivée d'une cellule pouvant être obtenue par la méthode de la revendication 9.

12. Plasmide pCGN1401 (ATCC 67227).
